# EUROPEAN PATENT SPECIFICATION

(11) **EP 1 428 647 B1**
(45) Date of publication and mention of the grant of the patent: **14.03.2012**
(21) Application number: 02080205.4
(22) Date of filing: 10.12.2002
(51) Int. Cl.: B29C 65/08, A61F 5/448

(54) **The ultrasonic welding of a coupling member onto an integrated top layer of polymeric foam of a stoma body side wafer for a two-piece stoma system**
Das Schweissen eines Kupplungselementes mittels Ultraschall auf eine integrierte obere Polymerschaumschicht einer körperseitigen Scheibe für ein zweiteiliges Ostomiesystem
Le soudage par ultrasons d'un élément d'accouplement sur une couche supérieure en mousse polymérique intégrée dans une plaquette coté corp pour un système d'ostomie en deux parties

(43) Date of publication of application: 16.06.2004
(73) Proprietor: Eurotec Beheer B.V., 4705 AG Roosendaal (NL)
(72) Inventor: Gijsbert van der Leden, Arie, 2950 Kapellen (BE)
(74) Representative: Groot Koerkamp, Jasper Henri

(56) References cited:
- EP-A- 0 435 638
- EP-A- 0 927 549
- GB-A- 1 252 940
- GB-A- 1 571 657
- JP-A- 62 227 725
- US-A- 4 572 753
- US-A- 4 770 730
- PATENT ABSTRACTS OF JAPAN vol. 007, no. 120 (M-217), 25 May 1983 (1983-05-25) & JP 58 038130 A (MITSUI TOATSU KAGAKU KK), 5 March 1983 (1983-03-05)

## Description

A number of diseases involving the intestinal and urinary tracts can result in the construction of an artificial outlet on the abdominal area for stool and/or urine; this is referred to as a stoma.

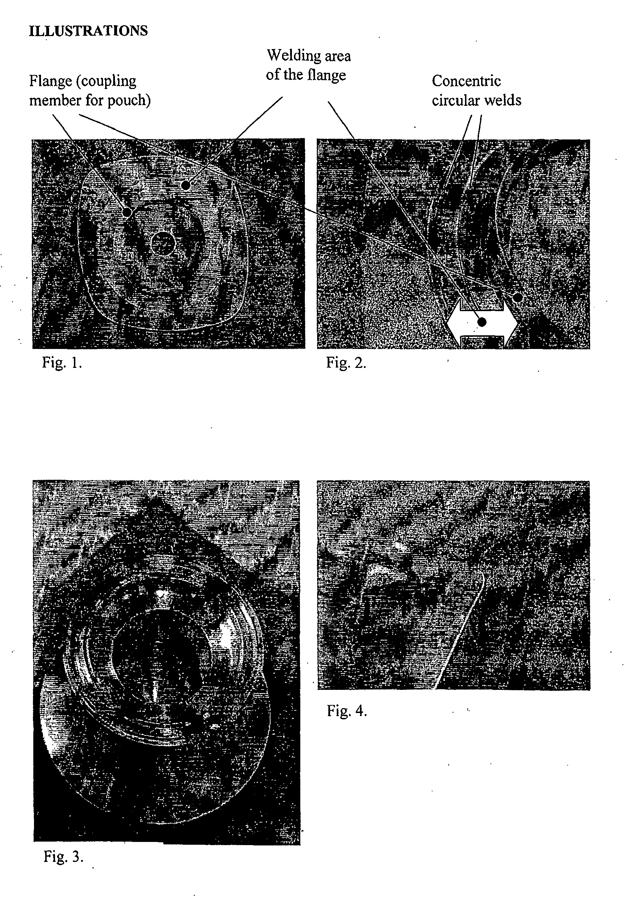

A diversion of the large intestine into the abdominal wall is referred to as a colostomy. The diversion of the small intestine is referred to as an ileostomy and when urine is disposed of through an artificial opening in the abdominal wall one refers to a urostomy. Usually a stoma patient is totally dependent on a well-functioning external collection device attached to the peristomal skin. Over the years a large variety of stoma appliances has been developed. Patients with a colostomy collect the relatively normal faeces in closed pouches which are usually changed several times a day. Patients with an ileostomy collect their relatively liquid stool in drainable pouches, the opening of which can be opened and closed with a tail clip. Patients with a urostomy collect their urine in a pouch with a drain tap. Ileostomy and urostomy pouches are usually changed once a day. Stoma appliances can be divided into two main systems; the 1-piece system and the 2-piece system. In the case of a 1-piece stoma system, the body side wafer, which consists of a special adhesive, is an integral part of the pouch. This type of pouch is designed to be fixed directly to the skin and has the advantage that the total surface of the adhesive layer is flat and flexible, which allows it to follow the contours of the skin easily. However, the major disadvantage of a 1-piece system is that the adhesive has to be removed from the skin with every pouch change, which may cause skin irritations. In the case of a 2-piece stoma system, the body side adhesive layer and the pouch are two separate components (see fig. 1; picture of a 2-piece body side wafer): First one attaches a special body side wafer with flange on to the skin. This flange is usually made from plastic and has a circular shape with an upstanding or projecting rib as a coupling member. The stoma pouch is also provided with a circular coupling member which can be attached on or around the projecting rib of the body side flange. A major advantage of a 2-piece stoma system is that the peristomal skin is not disturbed during pouch changes, as the adhesive part stays in place.

A major disadvantage of a 2-piece stoma system is that the body side adhesive wafer is far less flexible and flat compared with a 1-piece system because of the rigidity and the higher profile of the built-in coupling member, which makes it less suitable for stomas positioned in difficult areas such as folds and irregularities of the skin. The design of most 2-piece stoma systems is based on the 2-piece stoma system designed by Steer et al. (British patent application 571, 657). This stoma system consists of a body side wafer produced from a special hydrocolloid adhesive, covered by a thin polyethylene film, on which a circular polymeric flange with an upstanding or projecting rib has been attached which functions as a coupling member (see fig. 1 and 2). The 2-piece stoma system developed by Steer et al. is characterised by its property that all stoma appliances and stoma pouches within this system are provided with a polymeric coupling member with a circular channel, made to snap tightly over the projecting rib on the body side flanges. The projecting rib fits exactly in between the two walls of the channel in the coupling member of the stoma pouch. In order to be able to attach the coupling member (flange) firmly onto the body side wafer, the upper side of the body side wafer of the 2-piece stoma system designed by Steer et al. is laminated with a layer of polyethylene film. The major disadvantage of this polyethylene film is that it makes the body side wafer rigid and less flexible. The advantage of this polyethylene film is that is allows easy welding of the flange by means of an ultrasonic welding technique, in particular where the composition of the polymeric flange is at least partly identical or chemically equivalent to the polyethylene top layer of the body side wafer. With this ultrasonic welding technique high-frequency sound waves are generated by a so-called sonotrode as a part of a special welding tool. These sound waves are intensified by means of a booster and transmitted to the parts to be welded with a metal horn (see fig. 3.) - most commonly made from titanium or aluminium - which is provided with a special welding profile. When these sound waves are generated with the exact amplitude and transmitted to the horn, the molecules of both the polymeric flange and the polyethylene film are brought to a state of high-frequency vibration and the energy generated in this way will melt the two polymeric parts completely together according to the special welding profile at the end of the horn. The result of this welding process can be brought to perfection by determining the right combination of the strength of the sound waves, the welding time and the pressure of the horn profile into the surfaces to be welded. Because the welding area of the polymeric flange is relatively thick and the polyethylene top layer relatively thin, the process of ultrasonic welding is a critical process. If the mix mentioned above generates too much energy, the welding profile of the horn will burn into one or both polymeric surfaces being welded. If the mix mentioned above generates too little energy, the two parts being welded will not melt together or not enough. In both cases the welding strength of the flange to the surface of the body side flange will be insufficient, thus creating an unreliable product which may cause leakage and may severely impair the well-being of the end user. As described earlier, the disadvantage of the polyethylene top layer of the body side wafer of the 2-piece stoma system designed by Steer et al. is that it makes the body side wafer rigid and less flexible. For this reason softer polymeric flange materials with a lower profile were searched for on the one hand while on the other hand polymers were searched for which were able make the body side wafer more flexible, allowing the wafer to follow the contours of the skin much better. Replacement of the smooth polyethylene film with one with a waffle or a ribbed structure already helped to improve the flexibility of the body side wafer.

Replacement of the polyethylene top layer with another more flexible polymeric film, such as polyurethane film, creates an extremely flexible body side wafer, but does not allow ultrasonic welding of the flange. The use of a top layer of polyurethane foam or polyethylene foam also improves the flexibility of the 2-piece body side wafer. However it is almost impossible to weld polyurethane foam by means of ultrasonic welding. With the current technique it is not really possible to obtain a reliable seal when a relatively thick flange is welded ultrasonically on to a surface of polyethylene foam, because when the surface of the polymeric flange is melted together with the thin cell walls of the upper foam layer the traditional welding profile of the horn creates very little adhesion and the flange can therefore be easily torn away from the polymeric foam layer (see figure 4). Up till now this problem has been avoided by using other bonding techniques to attach a flange securely on a polymeric foam layer of a body side wafer. These methods are described below:

□ The flange is welded by heat sealing by applying heat locally on the welding area of the polymeric foam layer of the body side wafer. However this is not possible with the 2-piece stoma system of Steer et al., since the relatively thick welding area of the flange hinders the transmission of heat to the thin film on the body side wafer. In other words, the polymeric flange would already melt and deform before the heat could reach the polymeric foam or film layer to be welded on the body side wafer.

□ The flange is attached to the polymeric foam layer of the body side wafer by means of double-sided adhesive tape. This method, which is commonly used, consists of die-cutting circular adhesive rings from double-sided pressure sensitive adhesive tape which correspond in size with the welding area of the flange. When such a double-sided adhesive washer is pressed together between the polymeric foam layer of the body side wafer and the welding area of the flange, a reliable bond can be obtained. The major disadvantage of this method is that it is a labour-intensive and expensive process.

□ The flange is bonded to the polymeric foam layer of the body side wafer with the use of glue, usually a so-called 'hot melt'. This heat-activated glue is manually or automatically applied between the flange and the appropriate surface of the body side wafer and when the two parts are pressed together - and in case of a hot melt, after cooling down - a good bond is be obtained. The major disadvantage of this method is that manual bonding of the flange is very labour intensive and in the case of a more automated production method the bonding process, owing to the need for accurate dosage, is very complicated and requires high investments.

US patent 4,572,753 discloses a welding tip configuration for ultrasonically welding thermoplastic material including a plate with conical projections extending from a planar surface thereof and reservoirs recessed below the planar surface and spaced from the projections.

European patent application 0 927 549 discloses an adapter for a 2-piece stoma system, incorporating a new low profile coupling system, existing of two flat circular shaped coupling members of which the smaller coupling member has an aperture in the centre, whereby the inner circumference is completed with an inwardly extending deflectable seal strip and the larger coupling member has an aperture in the centre encircled by a projecting rib coupling member, whereby these two coupling members are joined together by a continuous relatively thing flexible polymeric wall. The 2-piece stoma system comprises a body side wafer comprising a polymeric top film to which a flange has been welded and a pouch having a pouch wall, which pouch wall has been provided with a channel shaped coupling member, which has been permanently affixed by means of a thermal-, HF- or ultrasonic welding technique. EP 0 927 549 forms the basis for the preamble of claim 1.

Japanese patent application JP 62 227725 A discloses a tool horn having two welding surfaces. A foam seated on a bearer and a skin laminated the foam are pressurised with the tool horn under the predetermined pressure or maximum pressurising force. Next, the tool horn is raised by operating a cylinder in order to reduce the pressurising force.

GB patent application GB 1 252 940 discloses a method of attaching a pad of spongy porous, thermoplastic material to a body of relatively rigid thermoplastic material comprising urging the pad and body into contact and applying ultrasonic energy of sufficient frequency and amplitude to a surface of the pad or body to secure the pad to the body.

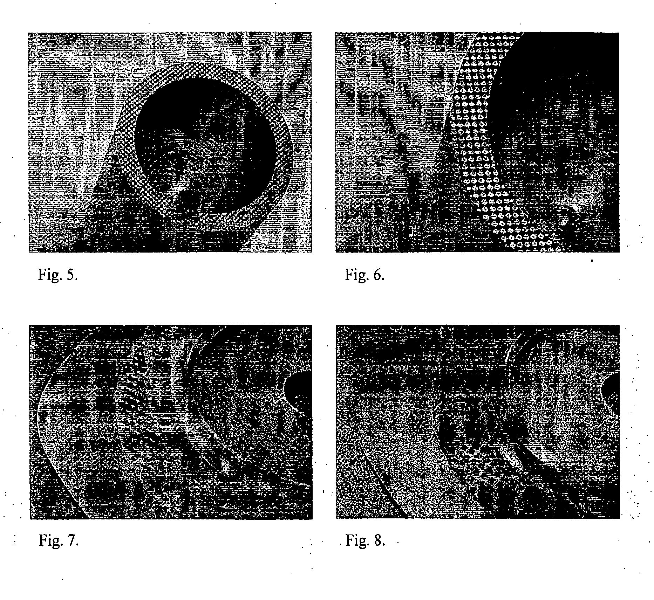

The invention of this patent application is based on a new method of ultrasonic welding of a flange on to a polymeric foam surface of a body side wafer of a 2-piece stoma system. For this purpose the horn is not provided with circular concentric slope line profiles, but the welding area is provided all over with some kind of a 'waffle profile'. The choice in this case is a pattern of linked mini-pyramids (see fig. 5 and 6), which are ultrasonically vibrated and under a relatively high pressure are pressed into the deeper area of the polymeric foam layer, during which process each individual mini-pyramid makes the polymeric walls of even deeper located layers of foam cells melt together according to the pattern of the horn. In this way a strong and reliable seal can be obtained over the full welding area between the flange and the polymeric foam layer so that the layers can not be easily torn apart. As this pattern is relatively easy to mill, a pattern of linked mini-pyramids was chosen in this instance (see fig. 6). However; for this process the welding profile could also consist of a pattern of other facet shapes or of small convex, trapezium, conical or cylindrical shapes.

Picture 1. Shows a commonly used body side wafer of a 2-piece stoma system with an integrated top layer of polyethylene film, where the welding area of the flange, which is the coupling member for the pouch, is welded together in the traditional way by using a horn with two protruding concentric circular welding profiles. With the correct combination of frequency and pressure, the flange and the polyethylene film are melted together underneath the concentric circular welding profiles.

Picture 2. Shows the concentric welds of the welding area of the flange described in picture I in more detail.

Picture 3. Shows the described welding profile of the traditional horn described in picture 1, in which the two protruding concentric welding circles can be easily seen.

Picture 4. Shows the poor results of an attempt to weld a flange on to a polymeric foam surface by the traditional ultrasonic method. The concentric circular welds have not melted together properly with the polymeric foam of the body side wafer and are furthermore sharply edged which means that the flange can easily be torn away resulting in an unreliable product.

Picture 5. Shows the special horn with a pattern of pyramid-shaped structures as described in this patent application.

Picture 6. Shows the welding profile of this special horn with a pattern of pyramid-shaped structures in greater detail.

Pictures 7 and 8. Show the results of the ultrasonic welding process with the special horn with a pattern of pyramid-shaped structures, where this pattern of the horn has caused the welding area of the flange not only to become melted together with the superficial cells of the foam layer, but also with the walls of the deeper located foam cells.

## Claims

1. Method of ultrasonic welding of a polymeric flange to a body side wafer for a 2-piece stoma system with a top layer of polymeric foam with a horn comprising a welding surface, **characterised in that** the welding surface comprises a pattern of protrusions.

2. Method of ultrasonic welding according to claim 1, wherein the protrusions have one of the following shapes:
a) A cone;
b) A pyramid;
c) A small convex;
d) A trapezium;
e) A cylinder; or
f) A flattened pyramid.

3. Method of ultrasonic welding according to claim 1, wherein the polymeric foam is polyurethane foam or polyethylene foam.

4. Method of ultrasonic welding according to claim 1, wherein the polymeric foam surface is a surface of a polymeric film.

## Patentansprüche

1. Verfahren für das Ultraschallschweißen eines polymeren Flansches an eine körperseitige Platte für ein 2-teiliges Stoma-System mit einer oberen Schicht aus Polymerschaumstoff mit einem eine Schweißfläche umfassenden Horn, **dadurch gekennzeichnet, dass** die Schweißfläche ein Muster von Vorsprüngen enthält.

2. Verfahren für das Ultraschallschweißen gemäß Anspruch 1, wobei die Vorsprünge eine der folgenden Formen haben:
a) einen Kegel;
b) eine Pyramide;
c) eine kleine Außenwölbung;
d) ein Trapez;
e) einen Zylinder oder
f) eine abgeflachte Pyramide.

3. Verfahren für das Ultraschallschweißen gemäß Anspruch 1, wobei es sich bei dem Polymerschaumstoff um Polyurethanschaumstoff oder Polyethylenschaumstoff handelt.

4. Verfahren für das Ultraschallschweißen gemäß Anspruch 1, wobei es sich bei der Polymerschaumstofffläche um eine Fläche einer Polymerfolie handelt.

## Revendications

1. Procédé de soudage par ultrasons d'une collerette polymère à une plaquette côté corps pour un système de stomie en deux pièces comportant une couche supérieure en mousse polymère avec une corne comprenant une surface de soudage, **caractérisée en ce que** la surface de soudage comporte une structure de saillies.

2. Procédé de soudage par ultrasons selon la revendication 1, dans lequel les saillies ont les formes suivantes :
a) un cône ;
b) une pyramide ;
c) une forme légèrement convexe ;
d) un trapèze ;
e) un cylindre ; ou
f) une pyramide aplatie.

3. Procédé de soudage par ultrasons selon la revendication 1, dans lequel la mousse polymère est une mousse en polyuréthane ou une mousse en polyéthylène.

4. Procédé de soudage par ultrasons selon la revendication 1, dans lequel la surface de la mousse polymère est une surface d'un film polymère.
